# EUROPEAN PATENT APPLICATION

(11) **EP 3 513 705 A1**
(43) Date of publication of application: **24.07.2019**
(21) Application number: 18152532.0
(22) Date of filing: 19.01.2018
(51) Int. Cl.: A61B 1/00, A61B 1/005, A61M 25/01

(54) **A METHOD FOR FIXATION OF A WIRE PORTION OF AN ENDOSCOPE, AND AN ENDOSCOPE**

(71) Applicant: Ambu A/S, 2750 Ballerup (DK)
(72) Inventor: LUND, Jesper Grøndahl, 2750 Ballerup (DK); HANSEN, Michael Kappler, 2665 Vallensbæk (DK)
(74) Representative: AWA Denmark A/S

(57) **Abstract**

A method for fixation of a wire portion of an endoscope comprising pulling a fourth wire portion so as to tension a steering wire, positioning second and third wire portions adjacent to each other, applying an adhesive on at least one of the second and third wire portions positioning a crimping member in a proximity of the second and third wire portions, and applying a crimping force to the crimping member so as to provide a crimp fixating the second and third wire portions in relation to each other, the crimp at least partly enclosing at least a portion of the adhesive. An endoscope with a steering wire having first, second, and third wire portions, an adhesive being provided on at least one surface of at least one of the second and third wire portions, and a crimped crimping member forming a crimp at least partly enclosing the second wire portion, the third wire portion, and at least a portion of the adhesive.

## Description

The present invention relates to a method for fixation of a wire portion of an endoscope for lung exploration and to an endoscope in which a tension of a steering wire is maintained.

Endoscopes are well known devices for visually inspecting parts of a body of a human or animal, which may be difficult to access, such as human body cavities. Typically, an endoscope comprises an elongated insertion tube with a handle at a proximal end as seen from an operator, and visual inspections means, such as a built-in camera, at a distal end of the elongated insertion tube. This definition of the terms distal and proximal, i.e. proximal being the end closest to the operator and distal being the end remote from the operator, as used herein for endoscopes in general, is adhered to in the present specification. Electrical wiring for the camera and other electronics such as LED lighting typically runs along an inside of the elongated insertion tube from the handle to the tip at the distal end. Instead of using cameras, endoscopes may also be fibre-optic, in which case the optical fibres typically run along an inside of the elongated insertion tube. A working channel may run along the inside of the insertion tube from the handle to the tip, e.g. allowing liquid to be removed from the body cavity or allowing for insertion of surgical instruments or the like into the body cavity.

In order to be able to manoeuvre a camera or the like of the endoscope inside the body cavity, the distal end of the endoscope may, in addition to the camera, comprise a section with increased flexibility, specifically an articulated or bendable tip part allowing the operator to bend this section to thereby move the camera. Typically, manoeuvring is carried out by tensioning or slacking steering wires in a guide tube also running along the inside of the elongated insertion tube from the articulated tip part to a control element with an operating member in the handle in an arrangement commonly known as a Bowden cable, cf. Bowden's original patent US-A-609570.

The steering wire running along the inside of the guide tube in a Bowden cable arrangement normally extends with a predetermined length over either end, allowing an operating member to be attached to a free (proximal) end of the wire, and an operated member to be attached to the other free (distal) end. When the ends of the guide tube are held stationary, movement of the proximal end of the steering wire with respect to the guide tube is transmitted to the distal end as a corresponding movement of the distal end of the steering wire with respect to the guide tube, so as to affect a movement of the operated member. The fastening of the proximal end of the guide tube to the operating handle is generally achieved with mechanical means where the guide tube is clamped, terminated in a block member, or adhered to the operating handle.

In order for the operator to have a good and responsive experience controlling the endoscope, the amount of play experienced by the operator should be as small as possible. The amount of play may depend on many factors, including the tension and friction of the wire.

To achieve a suitable tension of the steering wire, the wire is usually maintained in a pre-tensioned state. However, if the maintained tension of the steering wire is too high, the steerable tip part may be non-straight, or mechanical parts of the endoscope may break. On the other hand, if the tension of the steering wire is too low, the steering wire will have too much play and be partly or wholly unresponsive to the control of the operator. Therefore, the tension of the steering wire is usually adjusted before fixation of the steering wire, and the fixation of the steering wire ensures that the tension is maintained.

When fixating the steering wire in an endoscope, it is desired to provide a crimp which can resist a sufficiently high detachment force. A detachment force is understood as the force attempting to break the attachment between the crimp and the wire by pulling a portion of the wire on one side of the crimp away from a portion of the wire on the other side of crimp. The yield detachment force is the detachment force required to break the attachment between the crimp and the wire. In the present specification, this is also denoted as the yield strength of the crimp.

It is known from the prior art to fixate the steering wire in an endoscope by crimping the steering wire to itself, for instance from WO 2016/188537 A1.

On this background, it is an object of the present invention to provide an improved endoscope, preferably a disposable endoscope, and an improved method for fixation of a wire portion of an endoscope. This object may be met by a first and a second aspect of the invention.

The first aspect of the invention relates to a method for fixation of a wire portion of an endoscope, the method comprising the steps of:
a) providing:
   - an operating handle;
   - an insertion tube with a proximal end and a distal end, and with a steerable tip part located at the distal end;
   - a control element movable in relation to the operating handle;
   - a steering wire having a first, a second, a third, and a fourth wire portion, the first wire portion being connected to the steerable tip part, the second wire portion being located between the first and third wire portions, the third wire portion being located between the second and fourth wire portions; and
   - a crimping member;
b) pulling the fourth wire portion so as to tension the steering wire;
c) positioning the second and third wire portions adjacent to each other;
d) applying an adhesive on at least one of the second and third wire portions;
e) positioning the crimping member in a proximity of the second and third wire portions; and
f) subsequent to steps c), d), and e), applying a crimping force to the crimping member so as to provide a crimp fixating the second and third wire portions in relation to each other, the crimp at least partly enclosing at least a portion of the adhesive.

The method according to the first aspect of the invention may alternatively be provided as a method for fixation of a wire portion in a set of parts for an endoscope, the method comprising the steps of:
a) providing:
   - an operating handle;
   - an insertion tube with a proximal end and a distal end, and with a steerable tip part located at the distal end;
   - a control element movable in relation to the operating handle;
   - a steering wire having a first, a second, a third, and a fourth wire portion, the first wire portion being connected to the steerable tip part, the second wire portion being located between the first and third wire portions, the third wire portion being located between the second and fourth wire portions; and
   - a crimping member;
b) pulling the fourth wire portion so as to tension the steering wire;
c) positioning the second and third wire portions adjacent to each other;
d) applying an adhesive on at least one of the second and third wire portions;
e) positioning the crimping member in a proximity of the second and third wire portions; and
f) subsequent to steps c) and d), applying a crimping force to the crimping member so as to provide a crimp fixating the second and third wire portions in relation to each other, the crimp at least partly enclosing at least a portion of the adhesive.

In the prior art, steel has typically been employed as the steering wire material. A steel steering wire can achieve an adequate crimp yield strength combined with a tolerable amount of friction. It has been realized that, among other factors, the yield strength of the crimp depends on the friction of the wire, wherein a high friction of the wire provides a crimp with a high yield strength. The surface friction of steel steering wires is typically high, which results in a high crimp yield strength. However, a steel steering wire introduces a risk of providing an electrical connection between the operating handle and the steerable tip part of the insertion tube.

It has also been realized that the amount of friction of the steering wire and, thus, the amount of play depends on the friction between the steering wire and the guide tube or the like in which the steering wire is guided. As the friction of the steering wire increases, the amount of play also increases. In order to achieve a smooth controlling of the steerable tip part, it has therefore been realized that the coefficient of friction of the steering wire should be low. However, if using a wire of lower surface friction, the yield strength of the crimp may be reduced potentially to an inadequate level.

Surprisingly, it has been found that the method according to the invention may significantly increase a yield detachment force of the crimp joint, even with the use of adhesives of poor shear adhesion. Therefore, the method according to the invention may allow the use of a wider range of steering wire materials, including polymer plastics. Furthermore, the same tensioning method may be used as for steel steering wires. Additionally, tests have shown that the endoscope manufactured according to the invention may be resistant to aging, resulting in a satisfactory shelf life.

The term "endoscope" may be defined as a device suitable for examination of natural and/or artificial body openings, e.g. for exploration of a lung cavity. Additionally, or alternatively, the term "endoscope" may be defined as a medical device.

The term "steering wire" may be defined as an elongated member suitable for the purpose of controlling a steerable tip part by means of a control element, potentially as forming part of a cord drive or a Bowden cable arrangement for this purpose. The steering wire may further be tensionable. The term "steering wire" may include one or more from the group consisting of: a line, a cord, a thread, a string, a rope, a wire rope, a stranded wire rope, a cable, and a fishing line. Additionally or alternatively, the steering wire may be a monostranded, monofilament, multistranded or multifilament wire. A multistranded wire may also be known as a wire rope. In case of a multistranded wire, the strands may be braided, twisted, woven, coiled, or coiled wound.

The steering wire may comprise a metal, potentially steel. The steering wire may comprise, preferably consist essentially of, a polymer or plastic polymer or a combination of polymers suitable for being used as a wire. Alternatively or additionally, the steering wire may comprise one or more materials selected from the group consisting of: metal, steel, carbon steel, non-alloy carbon steel, non-alloy carbon steel with a carbon content of 0.3% to 1%, a polymer, a plastic polymer, polyethylene (PE), polyamide (PA), polyamide-imides (PAI), ultra-high-molecular-weight polyethylene (UHMWPE), high-density polyethylene (HDPE), low-density polyethylene (LDPE), high-molecular-weight polyethylene (HMWPE), natural fibres, artificial fibres, glass fibres, and carbon fibres. The steering wire may consist essentially of one or more of the above mentioned materials. In case of plastic polymers, such as PE, the fibres may be gel-spun. A plastic polymer may be defined as a synthetic malleable polymer, e.g. PE. Examples of suitable wires include wires comprising fibres traded under the trademarks Dyneema® and Honeywell Spectra®.

The material of the steering wire may have a dry static coefficient of friction lower than 0.5, 0.45 or 0.4. Additionally, or alternatively, the material of the steering wire may have a dry dynamic coefficient of friction lower than 0.4, 0.35 or 0.3. The coefficient of friction may be measured in relation to the same material.

The steering wire may be less than 1 mm, 0.75 mm, 0.60 mm, 0.40 mm or 0.30 mm in diameter.

The third and fourth wire portions of the steering wire may be coinciding with each other or may be one and the same wire portion. Alternatively, the third and fourth wire portions of the steering wire may be provided separately or located at a distance from each other.

During step b) of the method according to the first aspect of the invention, the wire may be tensioned to a first wire tension, and after step f) and/or step g) the tension of the wire may be maintained at a second wire tension. The first wire tension may be substantially the same as the second wire tension. Alternatively, the first and second wire tensions are different from each other.

In the endoscope assembled by the method according to the invention, the control element may be configured to allow an operator to control the steerable tip part of the insertion tube by the at least one steering wire. The control element may allow bending the steerable tip part in at least one direction, potentially in two directions, the two directions potentially being opposite. The control element may be accommodated in the operating handle. The control element may include a lever allowing an operator to control the control element. The lever may extend outwardly from the control element, potentially through the operating handle. The control element may be in the form of a roller or a roller disc.

The operating handle may be suitable for allowing an operator to grip and to operate the endoscope, potentially with one hand. The operating handle may comprise a handle housing arranged at a proximal end of the insertion tube. The handle housing may accommodate the control element.

The insertion tube or a distal end thereof may be suitable for insertion into a body cavity, potentially a lung, through a body opening, potentially a mouth. The body may be a natural and/or artificial body, potentially a human body. The insertion tube may extend from the operating handle towards a distal end of the endoscope.

The crimping member may be a potentially elongate and/or hollow member suitable for being crimped. The crimping member may have a tubular shape or a cylinder shell shape. The crimping member may comprise a spacing configured to receive a portion, potentially two portions, of a steering wire therein. An outer contour of a cross-sectional shape of the crimping member in a plane normal to the longitudinal axis thereof may be round or rounded, oval, triangular, ellipsoid, square, rectangular, polygonal, U-shaped; V-shaped, L-shaped, C-shaped or lens-shaped, wherein lens-shaped may be defined as the union of the intersection of two disks. The crimping member may be open or partially open, e.g. comprising a slit extending along the longitudinal axis, or it may be circumferentially closed.

A length of the crimping member may be at least 2 mm, 3 mm, 5 mm, 6 mm or 7 mm. A length of the crimping member may less than 30 mm, 25 mm, 20 mm, 17 mm or 15 mm. A length of the crimping member may be 2-30 mm, 3-25 mm, 5-20 mm, 6-17 mm or 7-15 mm.

The crimping member may comprise one or more materials selected from the group consisting of: metal, steel, aluminium, and titanium, polymer and plastic polymer. The crimping member may consist essentially of a material selected from this group of materials.

The term (noun) "crimp" may be defined as a portion of the crimping member which is deformed after applying the crimping force and/or as the crimping member after having been deformed. The crimp length may be defined as a length of the crimping member which has been crimped. The crimp length is potentially a fraction of the length of the crimping member.

The adhesive may be applied as a liquid adhesive, especially a glue, and/or may be a reactive adhesive and/or a chemically curing adhesive, and/or may be converted from a liquid state to a solid state from a chemical reaction. Such chemical reaction may be initiated by heat, moisture, radiation and/or pressure. The adhesive may be a single component adhesive selected from the group consisting of: anaerobic, cyanoacrylate, heat hardenable, moisture hardenable, radiation hardenable and silicone adhesive. The cyanoacrylate adhesive may be an ethyl 2-cyanoacrylate adhesive.

In the method according to the first aspect of the invention, step b) may comprise pulling the fourth wire portion so as to tension the steering wire potentially between the first and third wire portions.

Step c) may comprise positioning the steering wire around a wire guide so that the third wire portion is positioned adjacent to the second wire portion.

The second and third wire portions may be positioned adjacent to each other so that they extend substantially in parallel.

The second and third wire portions may be positioned so that they extend in opposite, potentially parallel, directions.

Step d) may comprise applying the adhesive on at least one of the second and third wire portions through an opening or through-hole, potentially a lateral opening or through-hole, of the crimping member.

Step d) may comprise applying the adhesive directly on at least one of the second and third wire portions.

Step d) may comprise applying the adhesive indirectly on at least one of the second and third wire portions, potentially by applying the adhesive to the crimping member, potentially to the inside of the crimping member, and then optionally positioning the crimping member in a proximity of the second and third wire portions.

Step d) may be performed before step e).

Step e) may comprise positioning the crimping member in a proximity of the second and third wire portions so that the crimping member at least partly encloses the second and third wire portions.

Step e) may comprise positioning the crimping member in a proximity of the second and third wire portions so that the crimping member abuts at least one of the second and third wire portions.

The term "partly enclosing" in step f) may alternatively be denoted as partly surrounding or partly covering.

Step f) may alternatively be defined as subsequent to steps c) and d), compressing the crimping member so as to provide a crimp fixating the second and third wire portions in relation to each other, the crimp at least partly enclosing at least a portion of the adhesive.

Step f) may comprise applying a crimping force to, and/or compressing the crimping member around at least a portion of the second and third wire portions so as to provide a crimp fixating the second and third wire portions in relation to each other, the crimp at least partly enclosing at least a portion of the adhesive.

Step f) may comprise positioning the crimping member between a first and a second tool part, and then moving the first and second tool part towards each other, whereby a crimping force is applied to the crimping member so as to provide a crimp fixating the second and third wire portions in relation to each other, the crimp at least partly enclosing at least a portion of the adhesive.

Step f) may comprise applying a crimping force to the crimping member by heating or cooling the crimping member so as to provide a crimp fixating the second and third wire portions in relation to each other, the crimp at least partly enclosing at least a portion of the adhesive. The crimping member may comprise a heat-shrinkable material or a cold-shrinkable material.

The crimp may fixate the second and third wire portions to each other and/or fixate the second and/or third wire portions to the crimping member.

In step e) and/or step f) the crimp may at least partly enclose the second and third wire portions.

The steps of the method according to the first aspect of the invention may be performed sequentially, potentially in the order a), b), c), d), e), f); in the order a), c), b), d), e), f); in the order a), b), c), e), d), f); or in the order a), c), b), e), d), f). However, the steps a) - e) are not necessarily performed in sequence, for instance step a) may be performed during step b) and/or step c).

In some embodiments of the method according to the first aspect of the invention, the adhesive is hardenable or settable, the method further comprising the step of: g) allowing the adhesive to harden or set. Preferably, step g) is performed after step f).

In some embodiments, the method further comprises the step of: h) releasing the fourth wire portion.

Step h) may be performed after step f), preferably after steps f)-g). Step h) may be performed after step a)-f), preferably after step a)-g). In step b) of the method, the pulling may be achieved by applying a pulling force to the fourth wire portion, and in step h) the pulling force may be released.

In some embodiments, the steering wire comprises at least two strands, wherein step d) of the method comprises applying the adhesive on at least one of the second and third wire portions so that the adhesive is distributed between at least two strands of the second and/or third wire portion(s). A "strand" may be defined as a wire strand. Additionally, or alternatively, a "strand" may be defined as a slender, threadlike line of material suitable for the purpose of being connected with other strands, potentially by braiding, twisting, weaving, coiling or coiled winding, to form a wire.

The steering wire may be a multistranded wire or wire rope. The wire may be braided, twisted, woven, coiled, or coiled wound. This may decrease elasticity of the steering wire, which may be desirable since it may reduce the amount of play experienced by an operator and may increase the fatigue resistance of the crimp, prolonging the working life of the endoscope.

Experiments show that the yield strength of a crimp is especially increased when the steering wire comprises strands. It is currently theorised that applying the adhesive so that the adhesive is distributed and hardened in between strands increases the local stiffness of the wire. This may ensure that, if the wire is subject to a detachment force, the local stiffness of the wire will increase the yield detachment force of the crimp, and thereby increase the yield strength of the crimp.

The steering wire may comprise at least 5, 10, 30, 100 or 1000 strands.

At least one, at least a quarter of, at least a majority of, all of or essentially all of the strands of the steering wire may comprise one or more materials selected from the group consisting of: metal, steel, polymer, plastic polymer, polyethylene (PE), polyamide (PA), polyamide-imides (PAI), ultra-high-molecular-weight polyethylene (UHMWPE), high-density polyethylene (HDPE), low-density polyethylene (LDPE), high-molecular-weight polyethylene (HMWPE), natural fibres, artificial fibres, glass fibres, and carbon fibres. At least one, at least a quarter of, a majority of or all of or essentially all of the strands may consist essentially of one or more of the above mentioned materials.

In some embodiments, the step d) of the method according to the first aspect of the invention comprises applying the adhesive as a liquid adhesive, optionally as a droplet, optionally on at least one of the second and third wire portions. This may have the advantage of increasing the stiffness of the wire, which in turn may increase the detachment force of the crimp joint.

Addtionally, or alternatively, step d) of the method comprises applying a volume of the adhesive as a liquid adhesive, optionally as a droplet, on at least one of the second and third wire portions, the volume optionally being 0.001 mL - 1 mL, 0.005 mL - 0.5 mL, 0.0075 mL - 0.25 mL or 0.01 mL - 0.1 mL.

In some embodiments, the step d) of the method according to the first aspect of the invention comprises distributing the adhesive on a length of at least one of the second and third wire portions, the length being equal to or above a diameter or a cross-sectional width of the steering wire. This may have the advantage of increasing the stiffness of the wire, which, in turn, may increase the detachment force of the crimp joint. Alternatively, or additionally, the length may be equal to at least a fifth of the crimp length, a quarter of crimp length, half the crimp length or the crimp length. Alternatively, or additionally, the length may be at least 1 mm, 2 mm, 3 mm, 4 mm or 5 mm.

In some embodiments, the step d) of the method according to the first aspect of the invention comprises at least applying the adhesive on the second and third wire portions at least partly between the second and third wire portions. This may have the advantage of increasing the stiffness of the wire, which, in turn, may increase the detachment force of the crimp joint. The step d) may comprise at least applying the adhesive on the second and third wire portions at least partly between the second and third wire portions.

In some embodiments, the step a) of the method according to the first aspect of the invention further comprises the step of:
providing a crimping tool comprising a first and a second tool part, the first tool part optionally including at least one protrusion, and/or the second tool optionally including at least one depression preferably corresponding to the at least one protrusion of the first tool part; and
wherein, optionally, in step e) the crimping tool further applies the crimping force by compressing, potentially opposite, sides of the crimping member so as to provide a crimp fixating the second and third wire portions in relation to each other, the crimp at least partly enclosing the second and third wire portions and at least a portion of the adhesive.

Step e) may comprise positioning the crimping member between the first and second tool parts, and then apply the crimping force by optionally moving the first and second tool part towards each other, whereby the protrusion of the first tool part is moved into a depression of the second tool part so as to provide a crimp fixating the second and third wire portions in relation to each other, the crimp at least partly enclosing the second and third wire portions and at least a portion of the adhesive.

In some embodiments, the steering wire comprises a polymer, potentially a plastic polymer. Hereby, the steering wire may comprise one or more materials selected from the group consisting of: polymer, plastic polymer, polyethylene (PE), polyamide (PA), polyamide-imides (PAI), ultra-high-molecular-weight polyethylene (UHMWPE), high-density polyethylene (HDPE), low-density polyethylene (LDPE) and high-molecular-weight polyethylene (HMWPE). The steering wire may consist essentially of one or more of the latter mentioned materials. An advantage of using a steering wire of polymer material may be that polymer materials, compared to steel, may have a lower electrical conductivity and may have a lower coefficient of friction. By using a steering wire with a low or no electrical conductivity, it is easier to provide a configuration of an endoscope that meets the desirable electrical safety requirements. By providing a lower coefficient of friction, the resistance to movement, when controlling the endoscope using the control element, may be reduced, which may ensure a smoother operation of the endoscope.

Using a polymer steering wire with a low coefficient of friction may present a challenge since steering wire is attached to itself by a crimp, which may essentially be defined as a friction joint, and the detachment force may generally decrease when reducing the friction coefficient. In spite of this, and as mentioned above, applying adhesive to the crimp joint has been shown to increase the detachment force of the crimp, especially when using a polymer steering wire, potentially to levels above a crimp with a steel steering wire without an adhesive, see further below. Especially in the case of stranded wires, it is currently theorised that this technical effect is achieved by increasing the stiffness of the portion of the steering wire located in the crimp so that an attempt to break the crimp joint by pulling a wire portion on one side of the crimp away from a wire portion on the other side of the crimp is resisted by having a relatively stiff wire portion which does not easily flex to fit a crimp deformity of the crimp.

The second aspect of the invention relates to an endoscope comprising:
- an operating handle;
- an insertion tube with a proximal end and a distal end, and with a steerable tip part located at the distal end;
- a control element movable in relation to the operating handle;
- a steering wire having first, second, and third wire portions, the first wire portion being connected to the steerable tip part, the second wire portion being located between the first and third wire portions;
- an adhesive provided on at least one surface of at least one of the second and third wire portions; and
- a crimped crimping member forming a crimp at least partly enclosing the second wire portion, the third wire portion, and at least a portion of the adhesive.

The crimp and the adhesive in conjunction may fixate or both contribute to the fixation of the second and third wire portions in relation to each other.

The endoscope according to the second aspect of the invention may be manufactured by means of the first aspect of the invention.

The endoscope according to the second aspect of the invention may alternatively be provided as a set of parts for an endoscope, the set of parts comprising:
- an operating handle;
- an insertion tube with a proximal end and a distal end, and with a steerable tip part located at the distal end;
- a control element movable in relation to the operating handle;
- a steering wire having first, second, and third wire portions, the first wire portion being connected to the steerable tip part, the second wire portion being located between the first and third wire portions;
- an adhesive provided on at least one surface of at least one of the second and third wire portions; and
- a crimped crimping member forming a crimp at least partly enclosing the second wire portion, the third wire portion, and at least a portion of the adhesive.

Preferably, the crimp and the adhesive in conjunction fixate or both contribute in the fixation the second and third wire portions in relation to each other.

In some embodiments of the endoscope according to the second aspect of the invention, the adhesive is provided so as to adhere:
- the crimp to at least one of the second and third wire portions; and/or
- the second and third wire portions to each other; and/or
- at least two strands of one of the second and third wire portions to each other; and/or
- the crimp to the second wire portion and/or the crimp to the third wire portion.

In some embodiments, the adhesive is a single component, anaerobic, cyanoacrylate, heat hardenable, moisture hardenable, radiation hardenable, and/or silicone adhesive.

In some embodiments, the crimp comprises at least one crimp deformity. A crimp deformity may be defined as the section of material deformed by a pair of a protrusion and a depression of a crimping tool. The crimp may comprise at least two, three or four crimp deformities. The crimping tool may comprise at least the same number of pairs of protrusions and depressions as the number of crimp deformities.

In some embodiments, the steering wire comprises a polymer, potentially a plastic polymer.

In some embodiments, the steering wire comprises at least two strands. The strands may be provided similarly as in the embodiments of the first aspect of the invention relating to strands, see above.

In some embodiments, two or more of the strands of the steering wire are braided and/or woven and/or twisted. This may decrease the elasticity of the steering wire, which may correspond to a reduction in elongation under the same loading. This is desirable since it reduces the amount of play experienced by an operator.

In some embodiments, at least part of the adhesive is located at least between at least two strands of at least one of the second and third wire portions. This may increase stiffness of steering wire in crimp and therefore increases detachment force required to break the crimp.

Any one or more of the embodiments relating to the first or second aspect of the invention may be combined with any one or more of the embodiments relating to either the same aspect of a different aspect.

In the following the invention will be described in more detail with reference to the drawings, in which:
Fig. 1 is a perspective view of an endoscope;
Fig. 2 is an exploded perspective view of the endoscope of Fig. 1 ;
Fig. 3 is a side view schematically illustrating operation of a steerable tip part of an endoscope;
Fig. 4a is a schematic view of a crimping tool;
Fig. 4b is a schematic cross-sectional view of a crimping tool;
Fig. 5 is a schematic view of a crimping tool and a crimping member prior to crimping with hidden lines shown as dashed lines;
Fig. 6 is a view similar to that of Fig. 5 showing the crimping member after crimping;
Fig. 7a is a schematic cross-sectional view of a crimping member after crimping;
Fig. 7b is another schematic cross-sectional view of the crimping member of Fig. 7a; and
Fig. 8 is a schematic view of a test setup for testing a yield detachment force of a crimp.

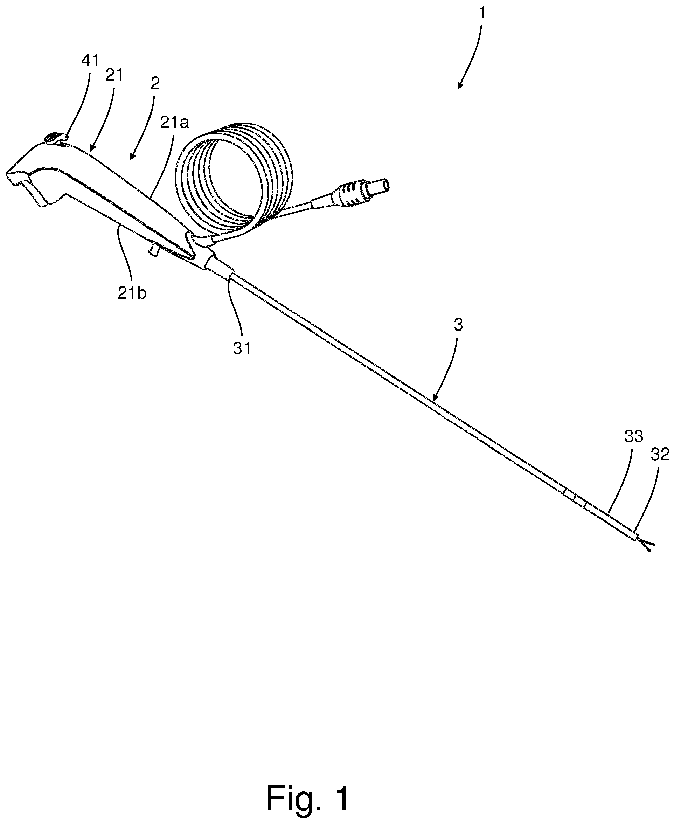

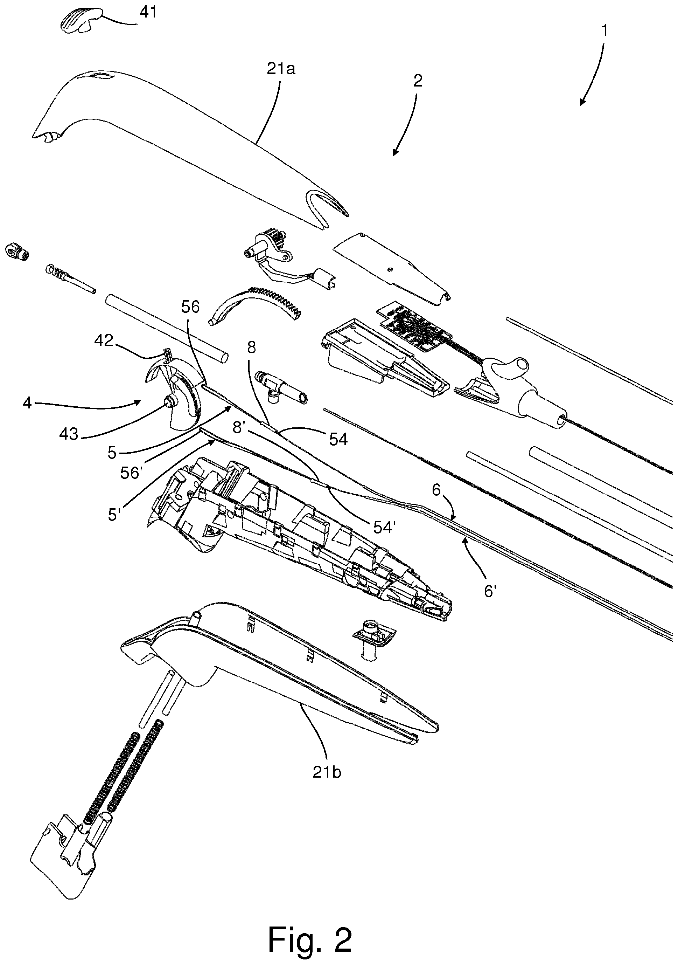

Fig. 1 shows an endoscope 1 according to an embodiment of the second aspect of the invention assembled by a method according to an embodiment of the second aspect of the invention. The endoscope is a medical device suitable for examination of natural and/or artificial body openings, e.g. for exploration of a lung cavity. The endoscope 1 comprises an operating handle 2, an insertion tube 3, and a control element 4, see also Fig. 2.

The operating handle 2 is a handle suitable for allowing an operator to grip and to operate the endoscope 1 with one hand. A handle housing 21, comprising two shells 21a, 21b, accommodates the control element 4.

The insertion tube 3 is an elongated member suitable for insertion into a patient, such as into a patient's lung through the patient's mouth. The insertion tube 3 extends from the operating handle 2 towards a distal end (to the right in Fig. 1) of the endoscope 1. The insertion tube 3 has a proximal end 31 connected to the handle housing and a distal end 32, and with a steerable tip part 33 located at the distal end 32.

The control element 4 is configured to allow an operator to control the steerable tip part 33 of the insertion tube 3 by two steering wires 5, 5', see also Fig. 2. The control element 4 allows bending the steerable tip part 33 in two directions. The control element 4 includes an operating member 41 allowing an operator to control the control element 4. The operating member 41 is connected to a lever 42 connected to and extending outwardly from the control element 4 through the handle housing 21 and is movable in relation to the operating handle 2.

Fig. 2 shows a set of parts for the endoscope 1 from Fig. 1, further showing that the endoscope comprises a first 5 and a second 5' steering wire each respectively located in a first 6 and a second wire support 6' in the form of a respective first and a second guide tube. The apostrophe suffix of a reference number denotes an element associated with the second steering wire 5' corresponding to a similar element associated with the first steering wire 5, e.g. the first wire support 6 is associated with the first steering wire 5, and the second wire support 6' is associated with the second steering wire 5'.

Each steering wire 5, 5' is an elongated, braided wire rope forming part of a Bowden cable arrangement for controlling the steerable tip part 33 by means of the control element 4. Each steering wire 5, 5' consists essentially of a plastic polymer in the form of ultra-high-molecular-weight polyethylene (UHMWPE) polymer. Each steering wire 5, 5' has a diameter of about 0.25 mm.

Each steering wire 5, 5' has a first (not shown); a second 52, 52'; a third 53, 53' and a fourth wire portion 54, 54'. Each of the first wire portions is connected to the steerable tip part 33. The wire portions are located in sequence first to third along each steering wire 5, 5' so that going from the first wire portion along the respective steering wire 5, 5', the next wire portion is the second wire portion 52, 52', then the third wire portion 53, 53', and lastly the fourth wire portion 54, 54', which terminates in a wire end.

Each steering wire 5, 5' extends from the second wire portion 52, 52', forms a loop 56, 56' and extends back in parallel to the second wire portion 52, 52' so that the second 52, 52' and third wire portions 53, 53' are located adjacently. An adhesive 7, 7' is applied on the second 52, 52' and third 53, 53' wire portions before the crimping action. Two crimping members 8, 8' enclose the respective second 52, 52' and third 53, 53' wire portions.

The control element 4 has two wire guides (not shown): a first wire guide for accommodating the loop 56 of the first steering wire 5, and a second wire guide for accommodating the loop 56' of the second steering wire 5'.

The lever 42 is attached to the control element 4 and allows an operator to rotate the control element 4 around an axis 43 in a known manner.

Each crimping member 8, 8' is an elongated and hollow member suitable for being crimped. Each crimping member 8, 8' has a cylinder shell shape with an internal spacing configured to receive two portions of a steering wire 5, 5'. An outer contour of the cross-sectional shape of each of the crimping members 8, 8' in a plane normal to the longitudinal axis thereof is round. The length of each of the crimping members 8, 8' is approximately 10 mm. Each of the crimping members 8, 8' essentially consists of steel.

The adhesive is a cyanoacrylate adhesive, in particular an ethyl 2-cyanoacrylate adhesive, which is a reactive single component adhesive, that, when chemically cured, converts from a liquid state to a solid state by a chemical reaction initiated by moisture, in particular air humidity. The chemical reaction is also known as hardening or setting.

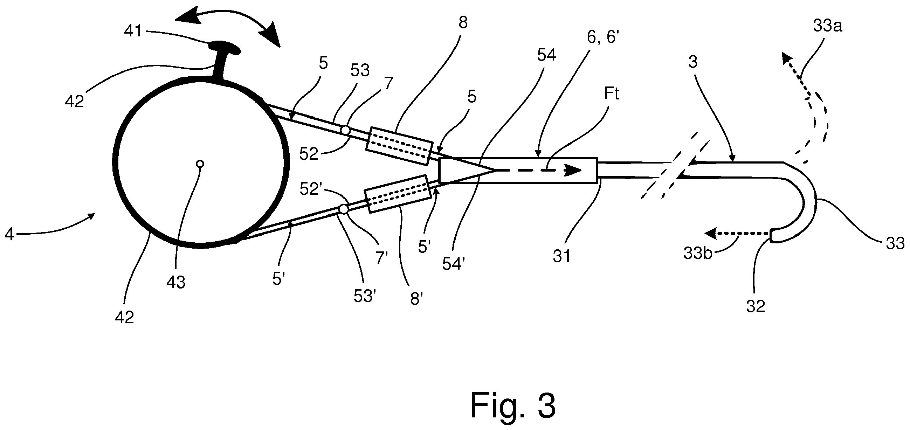

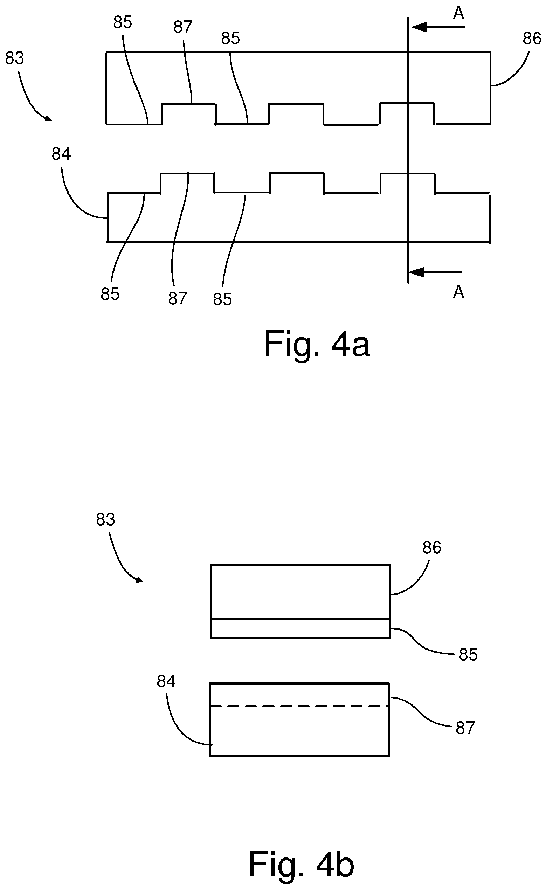

Figs 3-5 show an embodiment of the method according to the invention of attaching two steering wires 5, 5' in the endoscope 1 so as to allow control of a steerable tip part 33 by activation of the control element 4. The method can be provided in a similar fashion for an attaching a single steering wire. The guide tubes 6, 6' are schematically shown in Fig. 4 as a single guide tube 6, 6' which includes the two separate guide tubes 6, 6' as shown in Fig. 2.

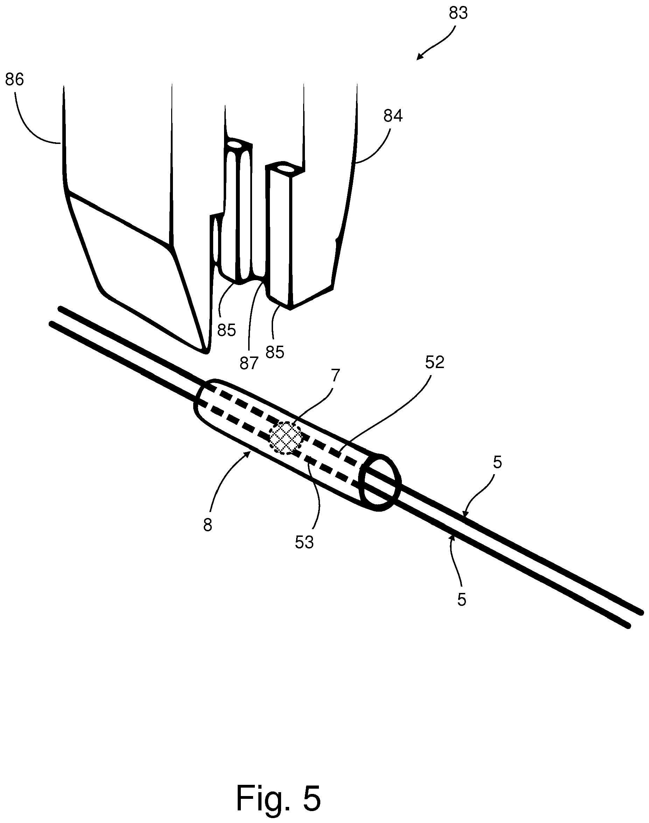

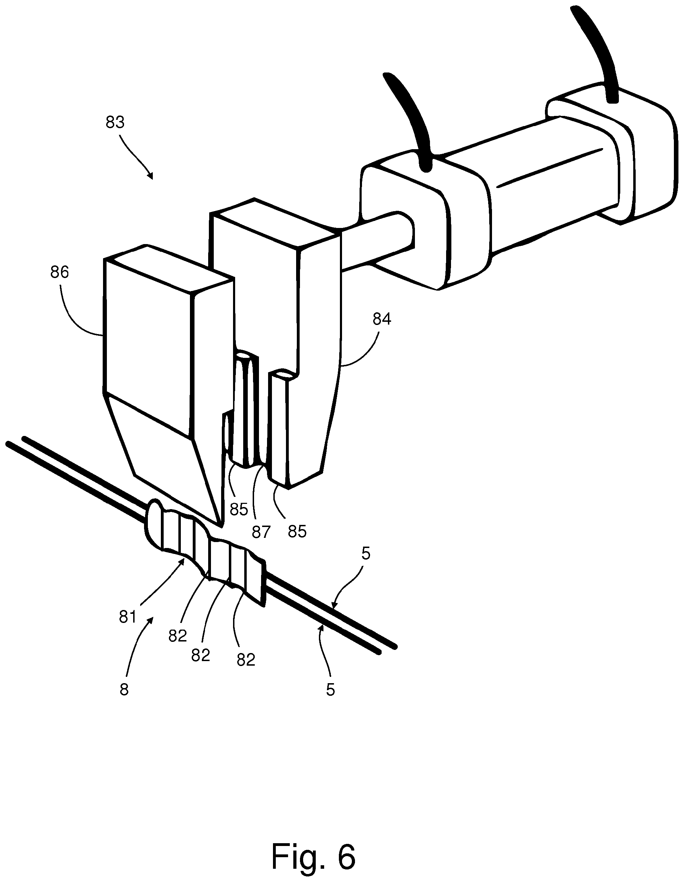

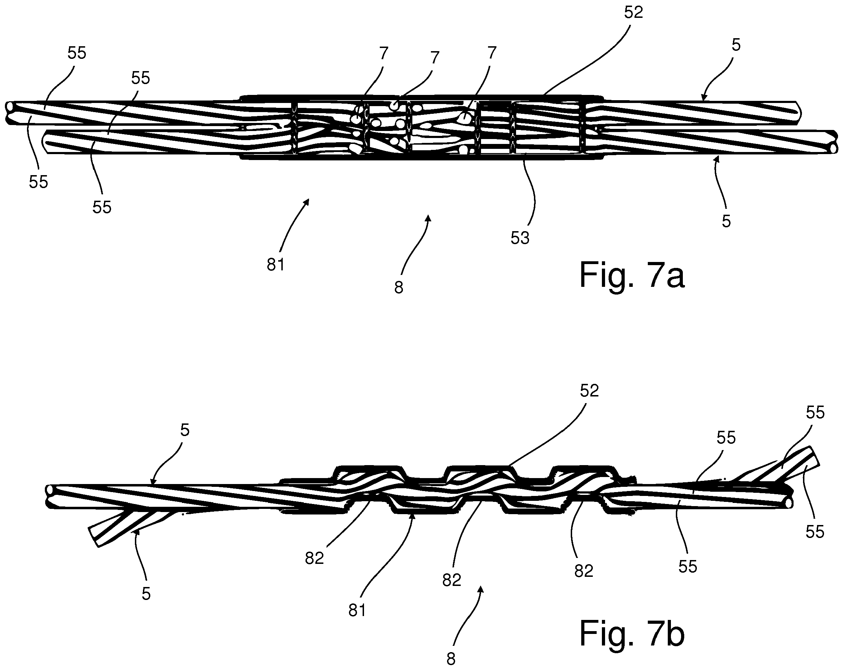

Referring to Fig. 3, the method of attaching two steering wires 5, 5' is carried out as follows:
First, the first steering wire 5 is attached to the steerable tip part 33 through the guide tube 6 so that, when pulled, the steerable tip part bends in a first direction 33a, which is shown in dashed lines in Fig. 3. The second steering wire 5' is attached to the steerable tip part 33 through the guide tube 6' so that, when pulled, the steerable tip part 33 bends in a second direction 33b, which is shown with solid lines in Fig. 3.
Second, the loops 56, 56' of the respective steering wires 5, 5' are positioned in their respective wire guides (not shown) in the control element 4. The second 52, 52' and third 53, 53' wire portions are then positioned adjacent to each other so that they extend in opposite and parallel directions.
Third, the fourth wire portions 54, 54' are pulled with a force Ft so as to tension the steering wires 5, 5' to a first wire tension between the first 51, 51' and the fourth wire portions 54, 54'. The tension of each wire is adjusted so that the steerable tip part 33 is straight.
Fourth, the adhesive 7, 7' is directly applied as a liquid adhesive droplet to a length of the second 52, 52' and third 53, 53' wire portions of each of the respective steering wires 5, 5'. This length is approximately four steering wire diameters or approximately 1 mm. The liquid adhesive is applied so that liquid adhesive is distributed between the strands 55, 55' of the second 52, 52' and third 53, 53' wire portions, which is shown in more detail in Figs 7a-b.
   A crimping tool 83 having a first tool part 84 and a second tool part 86 is positioned in a proximity to the crimping member 8, see Fig. 5. The crimping tool 83 has a plurality of pairs of corresponding protrusions 85 and depressions 87, wherein the first tool part 84 comprises one of the corresponding protrusions 85 and depressions 87 of a pair and the second tool part 86 comprises the other one of the corresponding protrusions 85 and depressions 87 of the pair, and wherein the plurality of pairs are located alternately across each tool part 84, 86 so that a protrusion 85 has adjacent depressions 87 on each side and a depression 87 has adjacent protrusions 85 on each side.
Fifth, as shown in Fig. 5 for a single crimping member 8, the crimping member 8 is positioned in proximity of the second 52 and third 53 wire portions so that it encloses the second 52 and third 53 wire portions and the adhesive 7.
Sixth, the crimping tool 83 is moved so that the crimping member 8 is positioned between the first 84 and second tool part 86. The first 84 and second 86 tool parts are then moved towards each other so that the tool parts 84, 86 abut the crimping member 8 and then moved further, thereby applying a crimping force to compress the crimping member 8 until the protrusion 85 of a pair protrude into the corresponding depression 87 of the pair so as to provide a crimp 81 fixating the second and third wire portions in relation to each other, see Fig. 6. The crimped crimping member 8 encloses the second 52 and third 53 wire portions and the adhesive 7. The crimp 81 has a plurality of crimp deformities 82, each formed by one pair of protrusion 85 and depression 87. This is seen in Fig. 6 for a single crimping member 8, a similar method may be applied for the second crimping member 8'.
   The crimp 81 is the portion of the crimping member 8 which is deformed after applying the crimping force.
Seventh, the adhesive 7 is allowed to harden so as to maintain the tension of the wire 5 in a second wire tension. The second wire tension is substantially the same as the first wire tension.

A crimping member 8 with a crimp 81 enclosing a second 52 and a third 53 wire portion of a single steering wire 5 manufactured according to the above method is shown in Figs 7a and 7b. The second wire portion 52 is positioned above the third wire portion 53, and droplets of the adhesive 7 are located and distributed between strands 55 of the steering wire inside the crimp. The adhesive 7 is positioned so that it adheres:
- the second 52 and third 53 wire portions to each other; and
- a plurality of strands of both the second 52 and third 53 wire portions to each other; and
- the crimp 81 to the second wire portion 52 and the crimp 81 to the third wire portion 53.

### Experiments

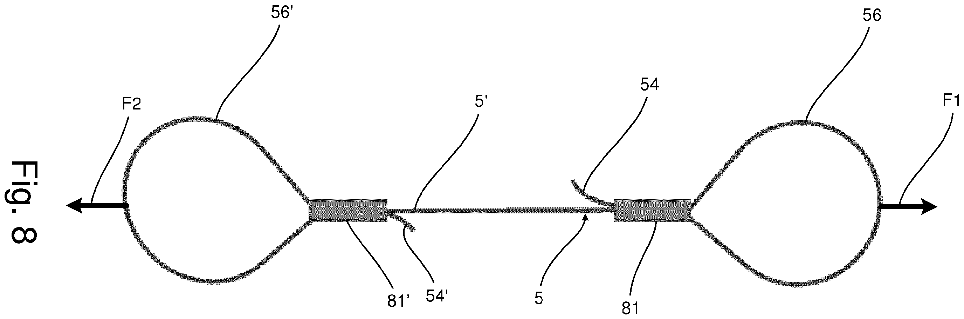

Experiments to measure the yield detachment force of a crimp were conducted. A number of test samples were provided as shown in Fig. 8. One test sample comprised a single steering wire with a right half 5 forming a right loop 56 defining a right eye and a left half 5' forming a left loop 56' defining a left eye. The right 5 and left 5' halves of the steering wire were crimped according to the method described in connection with Fig. 3 at a right crimp 81 and at a left crimp 81' applying cyanoacrylate as adhesive within the crimps 81, 81' as described above.

Test samples comprising a steel wire and a polymer wire were tested. Table 1 below describes the items used.

**Table 1: List of items**

| **Item** | **Description** |
|---|---|
| Steering wire - Steel | 402037, Ø0.25 |
| Steering wire - Polymer (UHMWPE) | RonThomsen - Hyperstrong, Ø0.25 |
| Crimping member | F75-10-M |
| | 888402043 |
| Adhesive | Cyanoacrylate (CA) glue |
| | Dana Lim A/S, Gelé 359 |

The yield detachment force of the crimp and wire was measured by applying a first pulling force F1 to the right eye of the test sample and a second pulling force F2 to the left eye of the test sample. The first F1 and second F2 pulling forces extended in parallel and opposite directions. The speed used to pull the test sample apart was 25 mm/min. The detachment force was then measured.

Four experiments were conducted with 8-12 repetitions for each experiment. The yield detachment force in Newtons (N) for each repetition in these experiments are listed in Tables 2 and 3 below. Each row corresponds to a single repetition of the respective experiment. The bottom row is the average result of all repetitions for that experiment.

The variables in the experiments are whether adhesive is applied or not and the material of the steering wire.

The first and second experiments shown in Table 2 were conducted using a steel steering wire, wherein the first experiment is the steel wire 5, 5' without adhesive in the crimps 81, 81', and wherein the second experiment is the same type of steel wire with hardened cyanoacrylate adhesive in the crimps 81, 81'.

**Table 2: Columns 1 and 2 relate to the experiments with a steel wire and without adhesive in the crimp. Columns 3 and 4 relate to the experiments with a steel wire and with cyanoacrylate (CA) adhesive in the crimp.**

| **ID** | **without CA glue [N]** | **ID** | **with CA glue [N]** |
|---|---|---|---|
| 1 | 45 | 1 | 59 |
| 2 | 43 | 2 | 57 |
| 3 | 45 | 3 | 57 |
| 4 | 55 | 4 | 57 |
| 5 | 44 | 5 | 57 |
| 6 | 28 | 6 | 58 |
| 7 | 28 | 7 | 56 |
| 8 | 33 | 8 | 58 |
| 9 | 53 | | |
| 10 | 38 | | |
| Average | 41 N | | 57 N |

The third and fourth experiments shown in Table 3 were conducted using a polymer steering wire, in particular a braided ultra-high-molecular-weight polyethylene (UHMWPE) wire rope. The third experiment is the same type of polymer wire rope 5, 5' without adhesive in the crimps 81, 81'. The fourth experiment is the same type of polymer wire rope 5, 5' as the previous experiment, this time with hardened cyanoacrylate adhesive in the crimps 81, 81'.

**Table 3: Columns 1 and 2 relate to the experiments with a braided UHMWPE wire rope and without adhesive in the crimp. Columns 3 and 4 relate to the experiments with the same type of braided UHMWPE wire rope and with cyanoacrylate (CA) adhesive in the crimps.**

| **ID** | **without CA glue [N]** | **ID** | **with CA glue [N]** |
|---|---|---|---|
| 1 | 18 | 1 | 55 |
| 2 | 16 | 2 | 60 |
| 3 | 17 | 3 | 64 |
| 4 | 19 | 4 | 65 |
| 5 | 18 | 5 | 60 |
| 6 | 17 | 6 | 56 |
| 7 | 21 | 7 | 58 |
| 8 | 16 | 8 | 64 |
| 9 | 15 | 9 | 59 |
| 10 | 19 | | |
| 11 | 24 | | |
| 12 | 17 | | |
| Average | 18 N | | 60 N |

In Table 4 below, the results are summarized. From these results it is seen that the yield detachment force is surprisingly increased when applying adhesive to the crimp. This effect is extremely pronounced for the polymer wire, where the experiments show that the yield detachment force more than triples when using the adhesive. In fact, the yield detachment force for the polymer wire using adhesive is greater than the yield detachment force of the steel wire, both with and without adhesive.

**Table 4: Yield detachment force increase in newtons in column 2, and in percentage in column 3, for the steel wire in row 2 and polymer wire in row 3. Results based on the experiments of Tables 2 and 3.**

| Steering wire material | Detachment force strength Increase with CA glue [N] | Detachment force strength Increase with CA glue [%] |
|---|---|---|
| Steel | +16N | +39% |
| Polymer | +42N | +233% |

### List of reference numerals

- 1: endoscope
- 2: operating handle
- 21: handle housing
- 3: insertion tube
- 31: proximal end
- 32: distal end
- 33: steerable tip part
- 33a: first direction
- 33b: second direction
- 4: control element
- 41: operating member
- 42: lever
- 43: axis
- 5: steering wire
- 51: first wire portion
- 52: second wire portion
- 53: third wire portion
- 54: fourth wire portion
- 55: strand
- 56: loop
- 6: wire support
- 7: adhesive
- 8: crimping member
- 81: crimp
- 82: crimp deformity
- 83: crimping tool
- 84: male tool part
- 85: protrusion
- 86: female tool part
- 87: depression

## Claims

1. A method for fixation of a wire portion of an endoscope, the method comprising the steps of:
a) providing:
- an operating handle;
- an insertion tube with a proximal end and a distal end, and with a steerable tip part located at the distal end;
- a control element movable in relation to the operating handle;
- a steering wire having a first, a second, a third and a fourth wire portion, the first wire portion being connected to the steerable tip part, the second wire portion being located between the first and third wire portions, the third wire portion being located between the second and fourth wire portions; and
- a crimping member;
b) pulling the fourth wire portion so as to tension the steering wire;
c) positioning the second and third wire portions adjacent to each other;
d) applying an adhesive on at least one of the second and third wire portions;
e) positioning the crimping member in a proximity of the second and third wire portions; and
f) subsequent to steps c), d), and e), applying a crimping force to the crimping member so as to provide a crimp fixating the second and third wire portions in relation to each other, the crimp at least partly enclosing at least a portion of the adhesive.

2. The method according to any of the previous claims, wherein the adhesive is hardenable or settable, the method further comprising the step of:
g) allowing the adhesive to harden or set.

3. The method according to any one of the previous claims, wherein the steering wire comprises at least two strands, and wherein step d) of the method comprises applying the adhesive on at least one of the second and third wire portions so that the adhesive is distributed between at least two strands of the second and/or third wire portion(s).

4. The method according to any one of the previous claims, wherein step d) of the method comprises applying the adhesive as a liquid adhesive on at least one of the second and third wire portions.

5. The method according to any one of the previous claims, wherein step d) comprises distributing the adhesive on a length of at least one of the second and third wire portions, the length being equal to or above a diameter or a cross-sectional width of the steering wire.

6. The method according to any one of the previous claims, wherein step d) comprises at least applying the adhesive on the second and third wire portions at least partly between the second and third wire portions.

7. The method according to any one of the previous claims, wherein step a) of the method further comprises the step of:
providing a crimping tool comprising a first and a second tool part, the first tool part including at least one protrusion, and/or the second tool including at least one depression preferably corresponding to the at least one protrusion of the first tool part; and wherein
in step e) the crimping tool applies the crimping force by compressing sides of the crimping member so as to provide a crimp fixating the second and third wire portions in relation to each other, the crimp at least partly enclosing the second and third wire portions and at least a portion of the adhesive.

8. The method according to any one of the previous claims, wherein the steering wire comprises a plastic polymer.

9. An endoscope, comprising:
- an operating handle;
- an insertion tube with a proximal end and a distal end, and with a steerable tip part located at the distal end;
- a control element movable in relation to the operating handle;
- a steering wire having first, second, and third wire portions, the first wire portion being connected to the steerable tip part, the second wire portion being located between the first and third wire portions;
- an adhesive provided on at least one surface of at least one of the second and third wire portions; and
- a crimping member forming a crimp at least partly enclosing the second wire portion, the third wire portion, and at least a portion of the adhesive.

10. The endoscope according to claim 9, wherein the adhesive is provided so as to adhere:
- the crimp to at least one of the second and third wire portions; and/or
- the second and third wire portions to each other; and/or
- at least two strands of one of the second and third wire portions to each other; and/or
- the crimp to the second wire portion and/or the crimp to the third wire portion.

11. The endoscope according to claim 9 or 10, wherein the adhesive is a single component, anaerobic, cyanoacrylate, heat hardenable, moisture hardenable, radiation hardenable and/or silicone adhesive.

12. The endoscope according to any one of claims 9 to 11, wherein the steering wire comprises a plastic polymer.

13. The endoscope according to any one of claims 9 to 12, wherein the steering wire comprises at least two strands.

14. The endoscope according to claim 13, wherein two or more of the strands of the steering wire are braided and/or woven and/or twisted.

15. The endoscope according to claim 13 or 14, wherein at least part of the adhesive is located at least between at least two strands of at least one of the second and third wire portions.
